# EUROPÄISCHE PATENTANMELDUNG

(11) **EP 3 791 836 A1**
(43) Veröffentlichungstag der Anmeldung: **17.03.2021**
(21) Anmeldenummer: 19196643.1
(22) Anmeldetag: 11.09.2019
(51) Int. Cl.: A61F 9/00

(54) **VORRICHTUNG ZUR INTRAVITREALEN INJEKTION IN EIN AUGE**

(71) Anmelder: Vitreoject UG (haftungsbeschränkt), 22085 Hamburg (DE)
(72) Erfinder: Kromer, Robert, 22085 Hamburg (DE)
(74) Vertreter: Uexküll & Stolberg

(57) **Zusammenfassung**

Eine Vorrichtung (2) zur intravitrealen Injektion in ein Auge, umfasst ein Gehäuse (4) mit einem proximalen Ende (5) und einem distalen Ende (6). Am proximalen Ende (5) des Gehäuses (4) ist eine erste Aufnahme (7) angeordnet, die ausgebildet ist, eine Injektionsnadel aufzunehmen, an der ein erster Spritzenkörper (10a) mit einem darin enthaltenen Medikament befestigbar ist. Am distalen Ende (6) des Gehäuses (4) ist eine umlaufende Anlagefläche (13) zur Anlage der Vorrichtung (2) an einen Bereich der Bindehaut des Auges vorgesehen. Die erste Aufnahme (7) ist in Richtung der Anlagefläche (13) derart verschiebbar, dass eine in der ersten Aufnahme (7) angeordnete Injektionsnadel in das Auge eindringen und das im ersten Spritzenkörper (10a) befindliche Medikament in das Auge injiziert werden kann.

Das Gehäuse (4) umfasst weiter eine Unterdruckkammer, die zum distalen Ende (6) des Gehäuses (4) hin derart geöffnet ist, dass ein in der Unterdruckkammer erzeugter Unterdruck die Anlagefläche (13) des Gehäuses (4) an das Auge ansaugt. Am proximalen Ende (5) des Gehäuses (4) ist eine zweite Aufnahme (8) für einen zweiten Spritzenkörper (10b) mit einem darin enthaltenem Fluid angeordnet. Die zweite Aufnahme (8) ist mit der Unterdruckkammer derart verbunden ist, dass sich das Fluid nach Abgabe aus einem in der zweiten Aufnahme (8) angeordneten zweiten Spritzenkörper (10b) innerhalb der Anlagefläche (13) auf dem Auge verteilt.

## Beschreibung

### Technisches Gebiet

Die vorliegende Erfindung betrifft eine Vorrichtung zur intravitrealen Injektion in ein Auge. Die Vorrichtung umfasst ein Gehäuse mit einem proximalen Ende und einem distalen Ende. Am proximalen Ende des Gehäuses ist eine erste Aufnahme angeordnet. Die erste Aufnahme ist ausgebildet, eine Injektionsnadel aufzunehmen, an der ein erster Spritzenkörper mit einem darin enthaltenen Medikament befestigbar ist. Am distalen Ende des Gehäuses ist eine umlaufende Anlagefläche zur Anlage der Vorrichtung an einen Bereich der Bindehaut des Auges vorgesehen. Die erste Aufnahme ist in Richtung der Anlagefläche derart verschiebbar, dass eine in der Aufnahme angeordnete Injektionsnadel in das Auge eindringen und das in dem ersten Spritzenkörper befindliche Medikament in das Auge injiziert werden kann.

### Hintergrund der Erfindung

Bei einer intravitrealen Medikamentenabgabe spritzt ein Augenarzt unter örtlicher Betäubung ein Medikament in das Innere des Augapfels. Dadurch gelangt der Wirkstoff des Medikaments direkt an die Netzhaut, wodurch Nebenwirkungen in den übrigen Organen vermieden werden. Besondere chemische Zusammensetzungen der Hüllsubstanzen für einige der Wirkstoffe ermöglichen darüber hinaus eine langsame und kontinuierliche Freisetzung des Wirkstoffs. So entfaltet sich die Wirkung nach und nach und hält über längere Zeiträume an.

Mithilfe der intravitrealen Injektion werden beispielsweise altersabhängige Makuladegenerationen und Netzhautschäden, die Folge einer langjährigen Zuckerkrankheit sind, behandelt. Eine dritte Indikation sind Venenverschlüsse, die zu einer Schwellung in der Netzhautmitte (Makulaödem) führen. Schließlich können Blutungen unter der Netzhaut, die diese abzuheben und zu schädigen drohen, mithilfe einer intravitrealen Injektion behandelt werden.

Um Infektionen des Auges, beispielsweise durch Bakterien von der Augenoberfläche, zu vermeiden, wird üblicherweise vor Beginn der Injektion eine Oberflächensterilisation durchgeführt. Bei einer solchen Sterilisation werden unter anderem jodhaltige pharmazeutische Erzeugnisse eingesetzt, um das Auge sowie den daran angrenzenden Bereich wie beispielsweise die Augenlider, die Wimpern und die Augenbrauen zu desinfizieren. Diese Sterilisationsmethode erfordert zusätzliche Verbrauchsmaterialien sowie einen erhöhten Zeitaufwand. Die damit verbundene längere Operationszeit kann das Risiko von Komplikationen erhöhen.

Eine Vorrichtung, die diesen Nachteil vermeidet, ist aus der EP 2 578 191 A1 bekannt. Die Medikamentenabgabevorrichtung ermöglicht eine lokale Desinfektion des Auges im Bereich der Einstichstelle einer Injektionsnadel. Die Medikamentenabgabevorrichtung umfasst einen Körper zur Aufnahme einer ein Medikament enthaltenen Patrone und ein mit dem Körper gekoppeltes Reservoir. Das Reservoir enthält ein Fluid, beispielsweise ein Desinfektionsmittel, und einen Kolben. Die Patrone ist mit einer Injektionsnadel verbunden und relativ zu dem Körper zwischen einer zurückgezogenen Position und einer ausgefahrenen Position axial bewegbar. Nach Aufsetzen der Vorrichtung auf die Bindehaut des Auges wird die Patrone axial von der zurückgezogenen Position in die ausgefahrende Position bewegt. Dadurch wird der Kolben ebenfalls axial verschoben und das Desinfektionsmittel aus dem Reservoir in Richtung des Auges gefördert. Das Desinfektionsmittel bedeckt den Einstichbereich der Injektionsnadel und desinfiziert diesen bevor die Injektionsnadel in das Auge eindringt.

Die Medikamentenabgabevorrichtung ist während der Injektion lediglich auf die Bindehaut aufgesetzt. Ein Verrutschen der Medikamentenabgabevorrichtung relativ zur Bindehaut ist möglich, weshalb eine ausreichende Sterilisation der Injektionsstelle nicht gewährleistet werden kann.

Die aus der anhängige europäische Patentanmeldung EP 19155658.8 bekannte Vorrichtung vermeidet diesen Nachteil. Die Vorrichtung hat ein Gehäuse, das eine Unterdruckkammer aufweist. Durch Anlegen eines Unterdrucks in der Unterdrucckammer wird das Gehäuse an das Auge angesaugt. Das Gehäuse umfasst weiter ein Reservoir, in dem ein Fluid angeordnet ist. Durch Anlegen eines Unterdrucks wird das Fluid aus dem Reservoir gefördert und innerhalb des Gehäuses auf dem Auge verteilt. Die Fertigung der Vorrichtung ist aufwendig und kostenintensiv.

Es ist daher Aufgabe der vorliegenden Erfindung, eine Vorrichtung zur intravitrealen Injektion in ein Auge bereitzustellen, die ein Verrutschen relativ zum Auge verhindert und kostengünstig herstellbar ist.

### Beschreibung der Erfindung

Diese Aufgabe wird gelöst durch eine Vorrichtung mit den Merkmalen gemäß Patentanspruch 1. Bevorzugte Ausführungsformen sind in den abhängigen Patentansprüchen beschrieben.

Gemäß der vorliegenden Erfindung umfasst das Gehäuse eine Unterdruckkammer, die zum distalen Ende des Gehäuses hin derart geöffnet ist, dass ein in der Unterdruckkammer erzeugter Unterdruck die Anlagefläche des Gehäuses an das Auge ansaugt. Am proximalen Ende des Gehäuses ist eine zweite Aufnahme für einen zweiten Spritzenkörper mit einem darin enthaltenem Fluid angeordnet. Die zweite Aufnahme ist derart mit der Unterdrucckammer verbunden, dass sich das Fluid nach Abgabe aus einem in der zweiten Aufnahme angeordneten zweiten Spritzenkörper innerhalb der Anlagefläche auf dem Auge verteilt.

Mithilfe des Unterdrucks in der Unterdruckkammer wird die Vorrichtung auf der Bindehaut des Auges fixiert. Bevorzugt dient der Unterdruck als erste Fixierungshilfe bis zur Abgabe des Fluids. Während der Abgabe des Fluids und danach, insbesondere während der Injektion des Medikaments in das Auge, ist optional eine zusätzliche manuelle Fixierung des Gehäuses der Vorrichtung auf dem Auge notwendig. Insbesondere kann der Unterdruck in der Unterdruckkammer nach Abgabe des Fluids aus einem in der zweiten Aufnahme angeordneten zweiten Spritzenkörper nicht mehr ausreichen, um das Gehäuse selbstständig auf der Bindehaut zu fixieren. Bevorzugt weist das Fluid desinfizierende und/oder anästhesierende Eigenschaften auf. Beispielsweise wird als Fluid PVD-Jod verwendet.

Die Vorrichtung kann als Einwegvorrichtung ausgebildet sein oder sich zur mehrmaligen Verwendung eignen. Soll die Vorrichtung mehrmals verwendet werden, ist sie derart ausgebildet, dass sie nach jedem Gebrauch sterilisieret werden kann. Bevorzugt wird die Vorrichtung in einer Verpackung steril vertrieben.

Im Rahmen der vorliegenden Erfindung wird unter dem Begriff "Unterdruck" ein Druck verstanden, der unter dem Umgebungsdruck von 1 bar liegt. Entsprechend wird im Rahmen der vorliegenden Erfindung unter dem Begriff "Unterdruckkammer" eine Kammer verstanden, in der der Umgebungsdruck von 1 bar reduziert werden kann und in einen Bereich zwischen 0 bar und 1 bar liegt.

Im Rahmen der vorliegenden Erfindung wird weiter unter dem Begriff "Spritzenkörper" ein hohlzylindrisch ausgebildeter Körper verstanden, an den eine Injektionsnadel (Kanüle) angesetzt und mit diesem verbunden werden kann. In einem Hohlraum des Spritzenkörpers ist ein Spritzenkolben verschiebbar angeordnet, der mittels eines rückseitig betätigbaren Stößels verschiebbar ist. Der Hohlraum kann beispielsweise ein Medikament in flüssiger Form aufnehmen, das durch Betätigung des Stößels aus dem Hohlraum und damit aus dem Spritzenkörper hinausbefördert und mithilfe der Injektionsnadel in einen Körper injiziert werden kann.

In einer Ausführungsform der vorliegenden Erfindung weist die Unterdruckkammer einen flexibel, manuell verformbaren Wandbereich auf, wobei die Unterdruckkammer derart ausgebildet ist, dass durch Verformung des Wandbereichs das Volumen der Unterdruckkammer, ausgehend von einer ursprünglichen Größe, verkleinerbar ist und durch Rückverformung des Wandbereichs das Volumen der Unterdruckkammer die ursprüngliche Größe wieder annimmt. Beispielsweise weist das Gehäuse eine Verschiebeachse auf, entlang der die erste Aufnahme in Richtung der Anlagefläche verschiebbar ist. Bevorzugt weist der Wandbereich eine von der Verschiebeachse des Gehäuses radial nach außen konvex geformte Wandung auf. Beispielsweise wird der flexible Wandbereich von einem Arzt mit einer Hand umfasst und flexible verformt, so dass das Volumen der Unterdruckkammer relativ zu einer ursprünglichen Größe verkleinert wird. Bevorzugt wird die dabei aus der Unterdruckkammer entweichende Luft über eine innerhalb der Anlagefläche vorgesehene distale Öffnung der Unterdruckkammer bzw. des Gehäuses aus diesem hinaus befördert. Nach Ansetzen der Anlagefläche auf der Bindehaut des Auges löst der Arzt seine Umgreifung des flexiblen Wandbereichs zumindest teilweise, so dass sich dieser zumindest teilweise flexible rückverformt, um seine ursprüngliche Größe wieder anzunehmen, und dabei einen Unterdruck in der Unterdruckkammer zu erzeugen. Bevorzugt ist das Gehäuse und insbesondere der flexibel, manuell verformbare Wandbereich ergonomisch geformt. Dadurch wird eine sichere Handhabung der Vorrichtung ermöglicht.

In einer weiteren Ausführungsform der vorliegenden Erfindung weist das Gehäuse eine Verschiebeeinrichtung auf, mittels der die erste Aufnahme entlang der Verschiebeachse, die sich von dem proximalen Ende zum distalen Ende des Gehäuse hin erstreck, von einer Ruheposition in eine Injektionsposition verschiebbar geführt ist, wobei eine in der ersten Aufnahme angeordnete Injektionsnadel in der Ruheposition von dem Auge beabstandet und in der Injektionsposition in das Auge eingedrungen ist. Mithilfe der Verschiebeeinrichtung wird ein sicheres Bewegen der ersten Aufnahme und damit einer in der ersten Aufnahme angeordneten Injektionsnadel mit einem ersten Spritzenkörper von der Ruheposition in die Injektionsposition gewährleistet. Damit wird ein präzises Einstechen der Injektionsnadel in die Bindehaut des Auges gewährleistet. Optional weist das Gehäuse eine Begrenzung des Fahrwegs von der eingefahrenen in die ausgefahrene Position der Aufnahme auf. Bevorzugt wird die Vorrichtung von einem Arzt während des Verschiebens der ersten Aufnahme von der Ruheposition in die Injektionsposition manuell umfasst, um die Vorrichtung zusätzlich vor einem Verrutschen relativ zum Auge zu sichern.

Bevorzugt umfasst die Verschiebeeinrichtung eine faltenbalgartige Struktur. Eine faltenbalgartige Struktur ermöglicht eine kontrollierte Faltung einzelner Balgelemente und damit ein Verkürzen der Struktur während des Verschiebens der ersten Aufnahme von der Ruheposition in die Injektionsposition.

In einer weiteren Ausführungsform der Erfindung ist die faltenbalgartige Struktur als ziehharmonikaartiger Abschnitt des Gehäuses ausgebildet. Bevorzugt weist das Gehäuse in dem ziehharmonikaartigen Abschnitt eine ziehharmonikaartige Wandung auf. Beispielsweise kann die ziehharmonikaartige Wandung ein oder mehrere Querverstrebungen quer zur Verschiebeachse des Gehäuses aufweisen, um ein Kollabieren der Wandung zu verhindern. Die Querverstrebungen können ein oder mehrere Durchgangslöcher aufweisen, durch die die Injektionsnadel hindurchragt. Bevorzugt weist das Gehäuse eine zylindrische Vertiefung auf, in der der ziehharmonikaartige Abschnitt angeordnet ist. Die Vertiefung stabilisiert den ziehharmonikaartigen Abschnitt zusätzlich und verhindert ein unkontrolliertes Kippen desselben. Bevorzugt weist der ziehharmonikaartige Abschnitt einzelne übereinander faltbare Wandelemente auf. Optional ist der ziehharmonikaartige Abschnitt in der Injektionsposition vollständig zusammengedrückt. Bevorzugt liegen die einzelnen faltbaren Wandelemente in der Injektionsposition annähernd parallel aneinander. Dadurch wird eine Begrenzung des Fahrwegs der Injektionsnadel von der Ruheposition in die Injektionsposition der ersten Aufnahme gewährleistet. Dies ermöglicht eine präzise Eindringtiefe der Injektionsnadel in das Auge. Ein unsachgemäßes Eindringen der Injektionsnadel in das Auge, das heißt beispielsweise ein zu tiefes Eindringen oder ein schräges Eindringen, wird somit vermieden. Bevorzugt bildet der ziehharmonikaartige Abschnitt des Gehäuses einen Rückstellmechanismus zum zumindest teilweisen automatischen Verfahren der ersten Aufnahme von der Injektionsposition in die Ruheposition. Der ziehharmonikaartige Abschnitt kann optional eine Federsteifigkeit aufweisen, so dass beim Verfahren der ersten Aufnahme von der Ruheposition in die Injektionsposition eine Federkraft überwunden werden muss, die die erste Aufnahme beim Loslassen zurück in Richtung der Ruheposition drückt. Dadurch kann optional ein sicheres und schnelles Herausziehen der Injektionsnadel aus dem Auge gewährleistet werden.

In einer Ausführungsform der Erfindung weist die Unterdrucckammer an einer der Anlagenfläche gegenüberliegenden Seite eine Durchgangsöffnung auf, über die die Unterdruckkammer mit der ersten Aufnahme verbunden ist und, durch die die Injektionsnadel in der Ruheposition der ersten Aufnahme hindurch ragt. Die Durchgangsöffnung bewirkt eine Führung der Injektionsnadel in dem Gehäuse. So ist gewährleistet, dass die Injektionsnadel in etwa im rechten Winkel zur Bindehaut in das Auge eindringt.

In einer weiteren Ausführungsform der Erfindung ist das Gehäuse einstückig geformt und vorzugsweise aus einem Elastomer hergestellt ist. Bevorzugt wird das Gehäuse im Spritzgussverfahren hergestellt. Dabei wird optional ein einziges Material oder Materialgemisch verwendet, beispielsweise Silikonelastomere. Einerseits muss das Material ausreichend flexibel sein, um beispielsweise eine Verformung des Wandbereichs der Unterdruckkammer und/oder eine Verformung des ziehharmonikaartigen Abschnitts des Gehäuses zu ermöglichen. Andererseits muss das verwendete Material/Materialgenmisch eine ausreichende Festigkeit des Gehäuses gewährleisten, so dass sich das Gehäuse als Ganzes nicht bei der Abgabe des Fluids aus einem in die zweite Aufnahme eingesetzten zweiten Spritzenkörper oder beim Verschieben der ersten Aufnahme in die Injektionsposition ungewollt verformt.

Bevorzugt weist das das Gehäuse an dem distalen Ende eine Positionierungseinrichtung zur Positionierung der Vorrichtung auf der Bindehaut des Auges auf. Dadurch wird erreicht, dass der Arzt die Vorrichtung leicht und gezielt an der richtigen Position auf der Bindehaut des Auges positionieren kann. Dies gewährleistet eine sichere intravitreale Injektion in ausreichender Entfernung von relevanten intraokularen Strukturen.

In einer Ausführungsform, der Erfindung ist die Positionierungseinrichtung als Außenkontur am distalen Ende des Gehäuses ausgebildet, wobei die Außenkontur derart geformt ist, dass sie zumindest teilweise einer Außenkontur einer Iris nachempfunden ist. Dies ermöglicht eine einfache Positionierung der Vorrichtung auf der Bindehaut des Auges. Ist die Außenkontur des distalen Endes des Gehäuses zumindest teilweise einer Außenkontur einer Iris nachempfunden, so muss der Arzt zur richtigen Positionierung der Vorrichtung auf der Bindehaut des Auges lediglich die Außenkontur des Gehäuses mit der Außenkontur der Iris überlagern. Dadurch ist eine deutlich vereinfachte und schnellere Positionierung der Vorrichtung auf der Bindehaut des Auges gewährleistet.

In einer weiteren Ausführungsform der Erfindung ist die Positionierungseinrichtung als farbliche Markierung des Gehäuses ausgebildet. Beispielsweise kann die farbliche Markierung im Bereich der Anlagefläche vorgesehen sein und einen Bereich kennzeichnen, in dem das Gehäuse die Iris überlappen soll. Bevorzugt ist die Positionierungseinrichtung als farblich flächige Markierung auf dem Gehäuse ausgebildet. Alternativ kann die farblich flächige Markierung auch ein durchfärbter Bereich der Wandung des Gehäuses sein. Optional sind mehrere zur Verschiebeachse rotationssymmetrisch auf dem Gehäuse angeordnete farbliche Markierungen vorgesehen. Optional sind zwei um 180° um die Verschiebeachse gedrehte farbliche Markierungen auf dem Gehäuse vorgesehen.

Bevorzugt weist die Vorrichtung einen Einsatz auf, der in die erste Aufnahme des Gehäuses eingesetzt ist, wobei sich der Einsatz ausgehend von dem proximalen Ende zum distalen Ende des Gehäuses hin verjüngt. Optional wir die Injektionsnadel vom Arzt selbst vor Benutzung der Vorrichtung in die erste Aufnahme des Gehäuses eingesetzt. Der Einsatz gewährleistet dabei eine Führung der Nadel, so dass diese einfach und schnell in die erste Aufnahme eingesetzt werden kann ohne das Gehäuse zu beschädigen. Optional sind der Einsatz und das Gehäuse aus unterschiedlichen Materialien hergestellt. Bevorzugt werden zur Herstellung des Einsatzes unter anderem gehärtete Silikonelastomere, Duroplaste oder Thermoplaste verwendet. Bevorzugt wird die Vorrichtung zusammen mit einem in der ersten Aufnahme montiertem Einsatz vertrieben.

In einer Ausführungsform der Erfindung umfasst die erste Aufnahme eine umlaufende Wulst, die ausgebildet ist, mit einer an dem Einsatz vorgesehenen umlaufenden Nut derart zusammenzuwirken, dass der Einsatz in der ersten Aufnahme fixiert ist. Die formschlüssige Verbindung durch Wulst und Nut ist eine einfache uns sichere Möglichkeit, den Einsatz im Gehäuse zu fixieren.

In einer weiteren Ausführungsform der Erfindung umfasst die Vorrichtung weiter eine Injektionsnadel, die in der ersten Aufnahme angeordnet ist. Optional wird eine Injektionsnadel nach Fertigung des Gehäuses in die erste Aufnahme eingesetzt und dort kraftschlüssig fixiert. Die Vorrichtung wir zusammen mit der Injektionsnadel sterilisiert und verpackt. Vor Verwendung der Vorrichtung muss der Arzt lediglich die Vorrichtung mit der Injektionsnadel aus der Verpackung entnehmen und erste und zweite Spritzenkörper, die das zu verabreichenden Medikament bzw. das Fluid enthalten, in die erste bzw. zweite Aufnahme des Gehäuses einsetzen. Dadurch wir eine einfach Handhabung der Vorrichtung ermöglicht.

Die oben genannte Aufgabe wird ebenfalls durch ein Injektionssystem mit den Merkmalen des Patentanspruchs 14 gelöst.

Das Injektionssystem umfasst eine erfindungsgemäße Vorrichtung zur intravitrealen Injektion in ein Auge und eine Injektionsnadel, die in der ersten Aufnahme des Gehäuses angeordnet ist. Das Injektionssystem umfasst weiter einen ersten Spritzenkörper, der mir der Injektionsnadel verbunden ist, und einen zweiten Spritzenkörper, der in der zweiten Aufnahme des Gehäuses angeordnet ist. Optional wird das Injektionssystem als mehrteiliges Set vertrieben, so dass der zu behandelnde Arzt sämtliche benötigte Elemente des Injektionssystems direkt griffbereit hat. Somit kann das Injektionssystem ohne vorheriges Suchen nach benötigten Spritzenkörpern oder Injektionsnadeln schnell und kostengünstig einsatzbereit gemacht werden.

Die Erfindung wird nachfolgend anhand von in den Figuren dargestellten bevorzugten Ausführungsformen erläutert. Die darin dargestellten Besonderheiten können einzeln oder in Kombination verwendet werden, um bevorzugte Ausgestaltungen der Erfindung zu schaffen. Die beschriebenen Ausführungsformen stellen keine Einschränkung der Allgemeinheit des in den Ansprüchen definierten Gegenstands dar. Es zeigen:
- Fig. 1: eine Seitenansicht eines erfindungsgemäßen Injektionssystems mit einer Vorrichtung zur intravitrealen Injektion in eine Auge,
- Fig. 2: eine Schnittansicht des Injektionssystems in der X-Y-Ebene gemäß Figur 1,
- Fig. 3: eine Schnittansicht der Vorrichtung in der Y-Z-Ebene gemäß Figur 1,
- Fig. 4: eine schematische Darstellung eines Auges und einem an das Auge angesetzten distalen Ende des Gehäuses in einer Schnittansicht entlang der Linie A-A in Figur 3,
- Fig. 5: eine schematische Darstellung eines Auges und eines an das Auge angelegten distalen Endes eines Gehäuses gemäß einer zweiten Ausführungsform in einer Schnittansicht und
- Fig. 6: eine Schnittansicht einer zweiten Ausführungsform der erfindungsgemäßen Vorrichtung.

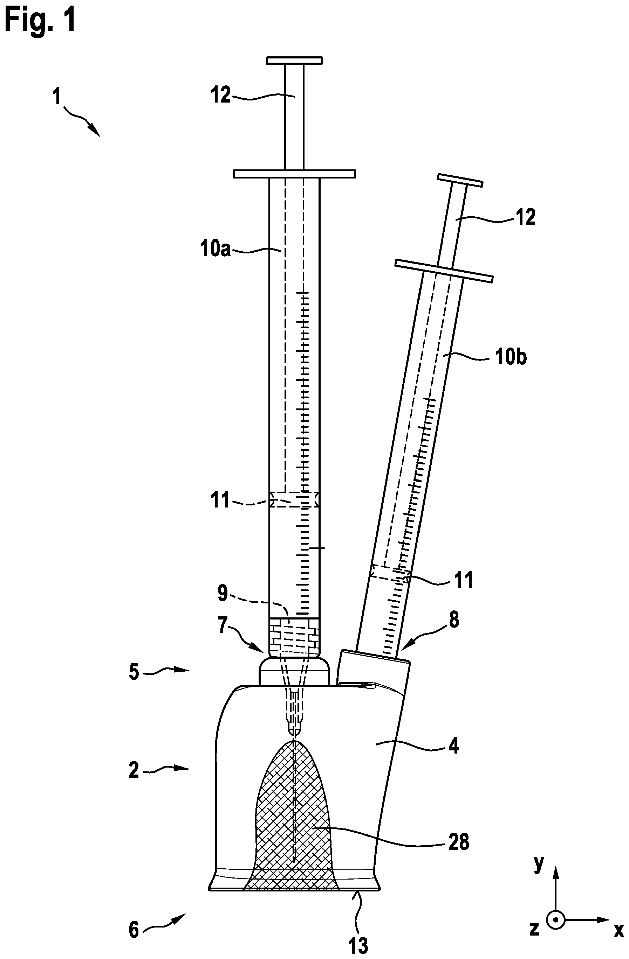

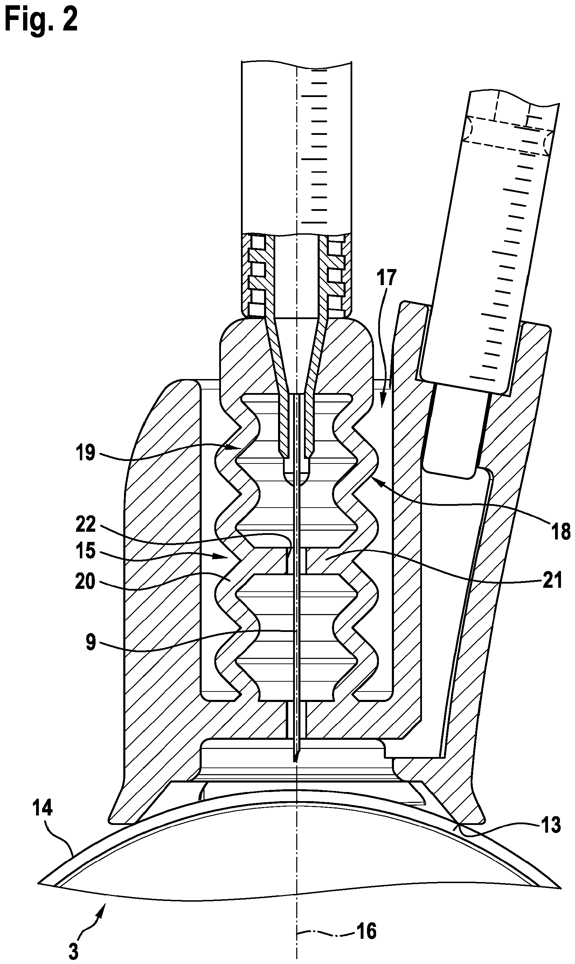

In den Figuren 1 und 2 ist ein Injektionssystem 1 umfassend eine Vorrichtung 2 zur intravitrealen Injektion in ein Auge 3 dargestellt. Die Vorrichtung 2 umfasst ein Gehäuse 4 mit einem proximalen Ende 5 und einem distalen Ende 6. Am proximalen Ende 5 des Gehäuses 5 sind eine erste Aufnahme 7 und eine zweite Aufnahme 8 angeordnet. Das Gehäuse ist einstückig mittels Spritzgießen geformt und vorzugsweise aus einem Elastomer hergestellt.

Die Vorrichtung 2 umfasst weiter eine Injektionsnadel 9, die in der ersten Aufnahme 7 angeordnet ist. An der Injektionsnadel 8 ist ein erster Spritzenkörper 10a des Injektionssystems 1 mit einem darin enthaltenen Medikament befestig. Das Injektionssystem 1 umfasst weiter einen zweiten Spritzenkörper 10b mit einem darin enthaltenem Fluid, der in der zweiten Aufnahme 8 des Gehäuses 4 angeordnet ist. Die Spritzenkörper 10a, 10b weisen jeweils einen Kolben 11 und einen Stößel 12 auf, mittels derer das Medikament bzw. das Fluid, beispielsweise Jod, aus dem jeweiligen Spritzenkörper 10a, 10b befördert werden kann.

Am distalen Ende 6 des Gehäuses 4 ist eine kreisförmige, umlaufende Anlagefläche 13 zur Anlage der Vorrichtung 2 an einen Bereich der Bindehaut 14 des Auges 3 vorgesehen.

Das Gehäuse weist eine Verschiebeeinrichtung 15 auf, mittels der die erste Aufnahme 7 entlang einer Verschiebeachse 16, die sich von dem proximalen Ende 5 zum distalen Ende 6 des Gehäuse 4 hin erstreck, von einer Ruheposition in eine Injektionsposition verschiebbar geführt ist. Die in der ersten Aufnahme 7 angeordnete Injektionsnadel 6 ist in der Ruheposition von dem Auge 3 beabstandet und in der Injektionsposition in das Auge 3 eingedrungen. Die erste Aufnahme 7 ist also in Richtung der Anlagefläche 13 derart verschiebbar, dass die in der ersten Aufnahme 7 angeordnete Injektionsnadel 9 in das Auge 3 eindringen und das im ersten Spritzenkörper 10a befindliche Medikament in das Auge 3 injiziert werden kann.

Die Verschiebeeinrichtung 16 ist in einer zylindrischen Vertiefung 17 des Gehäuses 4 angeordnet. Sie umfasst eine faltenbalgartige Struktur, die als ziehharmonikaartiger Abschnitt 18 des Gehäuses 4 ausgebildet ist. In dem ziehharmonikaartiger Abschnitt 18 weist das Gehäuse eine ziehharmonikaartige Wandung 19 mit übereinander faltbaren Wandelementen 20 auf. In der Injektionsposition liegen die faltbaren Wandelementen 20 annähernd parallel übereinander. Die ziehharmonikaartige Wandung 19 weist eine Querverstrebung 21 auf, die quer zur Verschiebeachse 16 des Gehäuses 4 angeordnet ist. Die Querverstrebung 21 umfasst ein Durchgangsloch 22, durch das die Injektionsnadel 9 ragt.

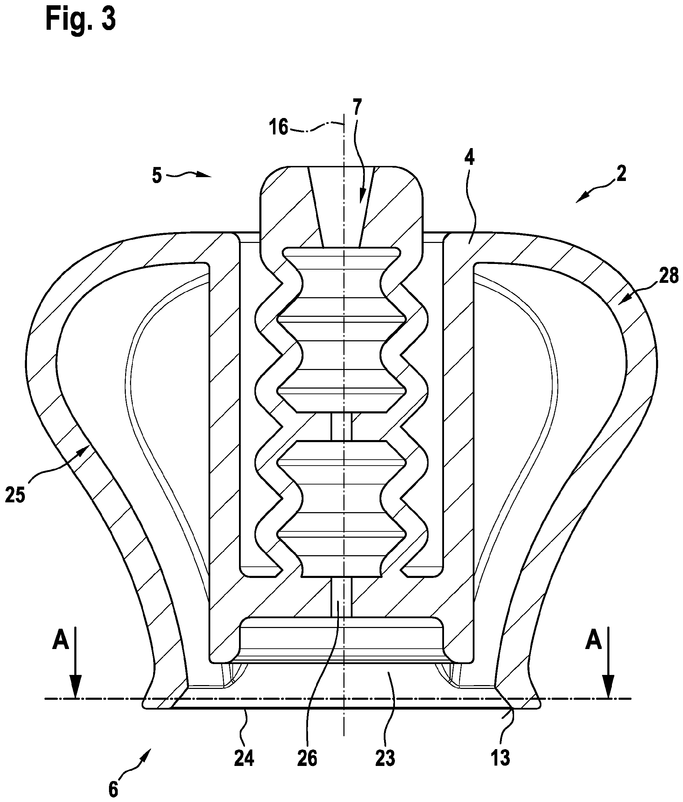

Wie in Figur 3 gezeigt ist, umfasst das Gehäuse 4 weiter eine Unterdruckkammer 23, die zum distalen Ende 6 des Gehäuses 4 hin eine Öffnung 24 hat. Die Unterdruckkammer 23 ist derart geöffnet, dass ein in der Unterdruckkammer 23 erzeugter Unterdruck die Anlagefläche 13 des Gehäuses 4 an das Auge 3 ansaugt. Die Unterdruckkammer 23 weist einen flexibel, manuell verformbaren Wandbereich 25 auf, wobei die Unterdruckkammer 23 derart ausgebildet ist, dass durch Verformung des Wandbereichs 25 das Volumen der Unterdruckkammer 23, ausgehend von einer ursprünglichen Größe, verkleinerbar ist und durch Rückverformung des Wandbereichs 25 das Volumen der Unterdruckkammer 23 die ursprüngliche Größe wieder annimmt.

Die Unterdruckkammer 23 hat an einer der Anlagenfläche 13 gegenüberliegenden Seite eine Durchgangsöffnung 26, über die die Unterdruckkammer 23 mit der ersten Aufnahme 7 verbunden ist und, durch die die Injektionsnadel 9 in der Ruheposition der ersten Aufnahme 7 hindurch ragt. Die zweite Aufnahme 8 ist mit der Unterdruckkammer 23 derart verbunden, dass sich das in dem zweiten Spritzenkörper 10b befindliche Fluid nach Abgabe aus dem zweiten Spritzenkörper 10b innerhalb der Anlagefläche 13 auf dem Auge 3 verteilt.

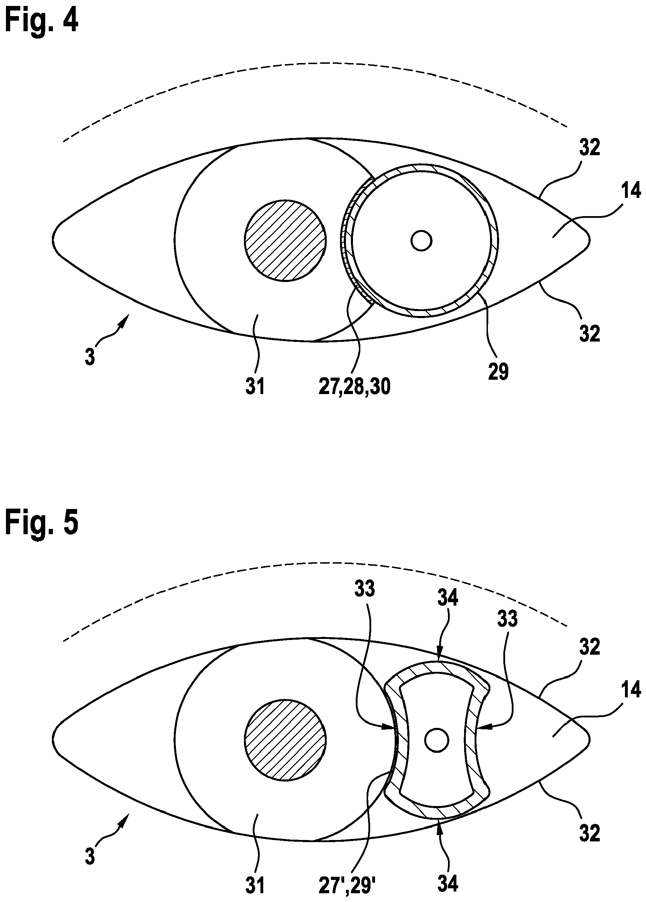

Figur 4 zeigt eine schematische Darstellung eines Auges 3 mit der Bindehaut 14 und einem auf die Bindehaut 14 aufgesetzten distalen Ende 6 des Gehäuses 4 der Vorrichtung 2 im Schnitt entlang der Linie A-A aus Figur 3.

Das Gehäuse 4 weist an seinem distalen Ende 6 eine Positionierungseinrichtung 27 zur Positionierung der Vorrichtung 2 auf der Bindehaut 14 des Auges 3. Die Positionierungseinrichtung 27 ist als farbliche Markierung 28 (Figur 1) auf dem Gehäuse 4 ausgebildet. Die farbliche Markierung 28 ist in Figur 4 zur vereinfachten Darstellung als zusätzliche Schicht 30 auf der Außenkontur 29 dargestellt. Alternativ kann das Gehäuse 4 im Bereich der farblichen Markierung 28 durchfärbt sein.

Die farbliche Markierung 28 ist auf die im Bereich des distalen Endes 6 kreisförmige Außenkontur 29 des Gehäuses 4 derart aufgebracht, dass sie einen Überlappungsbereich, in dem die Außenkontur 29 eine Iris 30 des Auges 3 überlappen soll, farblich markiert. Überlappt die farbliche Markierung 28 die Iris 31 und kommt die Außenkontur 29 des Gehäuses 4 an Augenlidern 32 des Auges 3 zur Anlage, so ist die Vorrichtung 2 und damit die Injektionsnadel 9 relative zum Auge 3 richtig positioniert.

Figur 5 zeigt eine zweite Ausführungsform der Positionierungseinrichtung 27 am distalen Ende 10 der Vorrichtung 2. Die Positionierungseinrichtung 27' unterscheidet sich von der in Figur 4 gezeigte dadurch, dass sie als Außenkontur 29' am distalen Ende 6 des Gehäuses 4 ausgebildet ist. Die Außenkontur 29' ist derart geformt, dass sie zumindest teilweise einer Außenkontur der Iris 31 des Auges 3 und zumindest teilweise einem Verlauf eines Übergangs zwischen den Augenlidern 32 und der Bindehaut 14 des Auges 3 nachempfunden ist. Die Außenkontur 29' umfasst zwei einander gegenüberliegen konkave Abschnitte 33, die der Außenkontur der Iris 31 nachempfunden sind, und zwei gegenüberliegende konvexe Abschnitte 34, die die konkaven Abschnitte 33 miteinander verbinden. Ist einer der konkaven Abschnitt 33 an den Verlauf der Iris 31 angelegt und kommen die konvexen Abschnitte 34 der Außenkontur 29' an den Augenlidern 32 des Auges 3 zur Anlage, so ist die Vorrichtung 2 und damit die Injektionsnadel 9 relative zum Auge 3 richtig positioniert.

Im Folgenden wird die Funktionsweise der Vorrichtung 2 unter Bezugnahme auf die Figuren 1 bis 5 erläutert:
Der Arzt entnimmt die Vorrichtung 1, aus einer sterilen Verpackung. Die Injektionsnadel 9 ist bereits in der ersten Aufnahme 7 angeordnet. Der Arzt zieht einen ersten Spritzenkörper 10a mit einem Medikament auf und befestigt den ersten Spritzenkörper 10a an der Injektionsnadel 9. Danach zieht der Arzt einen zweiten Spritzenkörper 10b mit einem Fluid, wie beispielsweise Jod, auf, und steckt den zweiten Spritzenkörper 10b in die zweite Aufnahme 8 des Gehäuses 4, so dass dieser in der zweiten Aufnahme 8 kraftschlüssig fixiert ist.

Nachfolgend umgreift der Arzt den flexiblen Wandbereich 25 der Unterdruckkammer 23 einhändig und verformt diesen manuell derart, dass das Volumen der Unterdruckkammer 23 verringert wird und die in der Unterdruckkammer 23 befindliche Luft durch die von der Anlagefläche 13 umgebenen Öffnung 24 zumindest teilweise entweicht. Die Anlagefläche 13 wird dann auf die Bindehaut 14 eines Auges 3 aufgesetzt und das Gehäuse 4 anhand der farbliche Markierung 28 oder anhand der Außenkontur 29' auf der Bindehaut 14 positioniert.

Lockert der Arzt seinen Griff um den flexiblen Wandbereich 25 der Vorrichtung 2, verformt sich dieser zumindest annähernd in seine Ausgangsform zurück. Da die Anlagefläche 13 annähernd luftdicht auf der Bindehaut 14 aufliegt, kann jedoch keine oder kaum Luft in die Unterdruckkammer 23 einströmen, so dass in der Unterdruckkammer 23 ein Unterdruck entsteht. Die Anlagefläche 13 saugt sich und damit die Vorrichtung 2 an der Bindehaut 14 fest.

Der Arzt hält die Vorrichtung 2 einhändig weiter fest, und betätigt den Stößel 12 des zweiten Spritzenkörper 10b derart, dass sich das darin befindliche Fluid in Richtung der Unterdruckkammer 23 bewegt und sich auf der Bindehaut 14 innerhalb der umlaufenden Anlagefläche 13 des Gehäuses 4 verteilt. Das Fluid desinfiziert bzw. anästhesiert die Bindehaut 14 im Bereich der Öffnung 24. Durch Betätigung des Stößels 12 verringert sich der Unterdruck in der Unterdruckkamme 23. Daher ist die einhändige Fixierung der Vorrichtung 2 durch den Arzt notwendig, um ein ungewolltes Verrutschen der Vorrichtung 2 auf der Bindehaut 124 zu verhindern.

Der Arzt verschiebt nun den ersten Spritzenkörper 10a zusammen mit der ersten Aufnahme 7 in Richtung der Anlagefläche 13 von der Ruheposition in die Injektionsposition. Dabei falten sich die faltbaren Wandelemente 30 übereinander, so dass diese annähend parallelen Wandelemente übereinanderliegen. Währender der Bewegung der ersten Aufnahme 7 von der Ruheposition in die Injektionsposition dringt die Injektionsnadel 9 in die Bindehaut 14 des Auges 3 ein. Liegen die faltbaren Wandelemente 30 annähernd parallel übereinander, ist die Injektionsposition und damit die maximale Eindringtiefe der Injektionsnadel 9 in das Auge 3 erreicht.

Ist die maximale Eindringtiefe der Injektionsnadel 9 erreicht, wird der Stößel 12 und damit der Kolben 11 relativ zum ersten Spritzenkörper 10a betätigt, so dass das im ersten Spritzenkörper 10a befindliche Medikament über die Injektionsnadel 9 in das Auge 3 injiziert wird.

Durch das Verschieben der ersten Aufnahme 7 in die Injektionsposition ist der Unterruck in der Unterdruckkammer 23 vollständig abgebaut und es herrscht Umgebungsdruck in der Unterdruckkammer 23.

Nach erfolgter Injektion des Medikaments wird die Vorrichtung 2 manuell vom Auge 20 abgehoben und damit die Injektionsnadel 9 aus dem Auge 3 herausgezogen. Da der Innendruck im Auge 3 höher ist als der Umgebungsdruck, wird die Eintrittswunde der Injektionsnadel 9 sofort nach deren Herausziehen aus dem Auge 3 verschlossen.

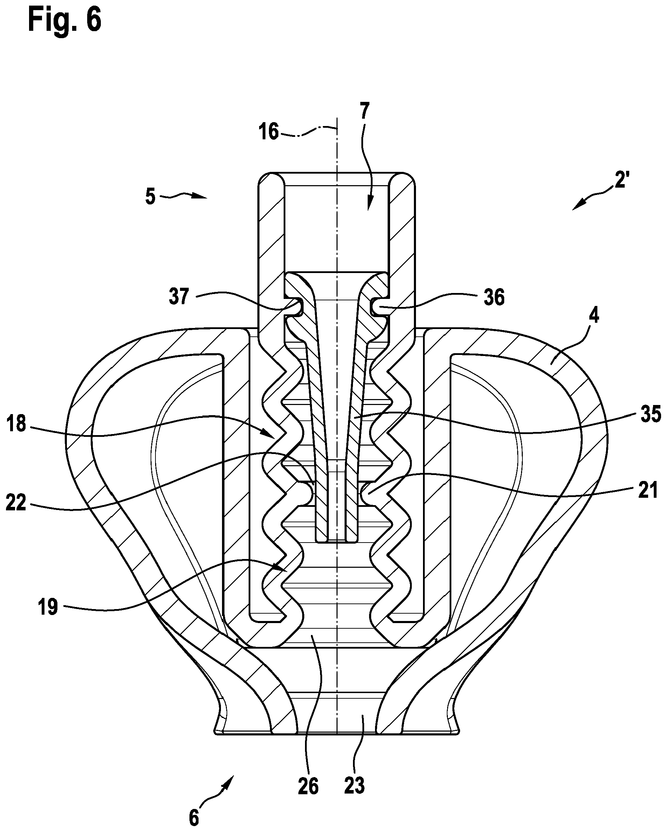

In Figur 6 ist eine zweite Ausführungsform der Vorrichtung 2 im Schnitt dargestellt. Die Vorrichtung 2' unterscheidet sich von der in den Figuren 1 bis 5 gezeigten dadurch, dass sie einen Einsatz 35 aufweist. Der Einsatz 35 ist in die erste Aufnahme 7 des Gehäuses 4 eingesetzt und verjüngt sich ausgehend von dem proximalen Ende 5 zum distalen Ende 6 des Gehäuses 4 hin. Der Einsatz 35 ragt in den zihharmonikaartigen Abschnitt 18 des Gehäuses 4 hinein und verläuft durch das Durchgangsloch 22 in der Querverstrebung 21 der ziehharmonikaartigen Wandung 19.

Die erste Aufnahme 7 umfasst eine umlaufende Wulst 36, die ausgebildet ist, mit einer an dem Einsatz 35 vorgesehenen umlaufenden Nut 37 derart zusammenzuwirken, dass der Einsatz 35 in der ersten Aufnahme 7 fixiert ist. Die erste Aufnahme 7 und der Einsatz 35 sind also formschlüssig miteinander verbunden. Im Vergleich zur Vorrichtung 2 weist die Vorrichtung 2' in der Unterdruckkammer 23 eine im Durchmesser größere Durchgangsöffnung 26 auf.

Die Vorrichtung 2' wird in einer sterilen Verpackung ohne Injektionsnadel 9 (Figur 2) vertrieben. Die Injektionsnadel 9 wir von dem behandelnden Arzt selbst in die erste Aufnahme 7 eingesetzt. Hierzu führt der Arzt die Injektionsnadel 9 durch die erste Aufnahme 7 in den Einsatz 35 ein und fixiert die Injektionsnadel 9 in der ersten Aufnahme 7 kraftschlüssig. Der Einsatz 35 führt die Injektionsnadel 9 derart, dass sie entlang der Verschiebeachse 16 ausgerichtet ist. Die weitere Funktionsweise der Vorrichtung 2' entspricht der Funktionsweise der Vorrichtung 2, wie sie unter Bezugnahme auf die Figuren 1 bis 5 oben erläutert wurde.

### Bezugszeichenliste

- 1: Injektionssystem
- 2,2': Vorrichtung
- 3: Auge
- 4: Gehäuse
- 5: proximales Ende (Gehäuse)
- 6: distales Ende (Gehäuse)
- 7: erste Aufnahme
- 8: zweite Aufnahme
- 9: Injektionsnadel
- 10a: erster Spritzenkörper
- 10b: zweiter Spritzenkörper
- 11: Kolben (Spritzenkörper)
- 12: Stößel (Spritzenkörper)
- 13: Anlagefläche
- 14: Bindehaut (Auge)
- 15: Verschiebeeinrichtung
- 16: Verschiebeachse
- 17: zylindrische Vertiefung
- 18: ziehharmonikaartiger Abschnitt (Gehäuse)
- 19: ziehharmonikaartige Wandung (Gehäuse)
- 20: Wandelement
- 21: Querverstrebung
- 22: Durchgangsloch (Querverstrebung)
- 23: Unterdruckkammer
- 24: Öffnung (Unterdruckkammer)
- 25: Wandbereich (Unterdruckkammer)
- 26: Durchgangsöffnung
- 27: Positionierungseinrichtung (Gehäuse)
- 28: farbliche Markierung (Positionierungseinrichtung)
- 29,29': Außenkontur (Gehäuse)
- 30: Schicht
- 31: Iris
- 32: Augenlider
- 33: konkaver Abschnitt
- 34: konvexer Abschnitt
- 35: Einsatz
- 36: Wulst
- 37: Nut

## Patentansprüche

1. Vorrichtung zur intravitrealen Injektion in ein Auge, umfassend:
ein Gehäuse (4) mit einem proximalen Ende (5) und einem distalen Ende (6),
wobei am proximalen Ende (5) des Gehäuses (4) eine erste Aufnahme (7) angeordnet ist, die ausgebildet ist, eine Injektionsnadel (9) aufzunehmen, an der ein erster Spritzenkörper (10a) mit einem darin enthaltenen Medikament befestigbar ist,
wobei am distalen Ende (6) des Gehäuses (4) eine umlaufende Anlagefläche (13) zur Anlage der Vorrichtung (2, 2') an einen Bereich der Bindehaut (14) des Auges (3) vorgesehen ist und,
wobei die erste Aufnahme (7) in Richtung der Anlagefläche (13) derart verschiebbar ist, dass eine in der ersten Aufnahme (7) angeordnete Injektionsnadel (9) in das Auge (3) eindringen und das in dem ersten Spritzenkörper (10a) befindliche Medikament in das Auge (3) injiziert werden kann,
**dadurch gekennzeichnet,**
**dass** das Gehäuse (4) weiter eine Unterdruckkammer (23) umfasst, die zum distalen Ende (6) des Gehäuses (4) hin derart geöffnet ist, dass ein in der Unterdruckkammer (23) erzeugter Unterdruck die Anlagefläche (13) des Gehäuses (4) an das Auge (3) ansaugt,
**dass** am proximalen Ende (5) des Gehäuses (4) eine zweite Aufnahme (8) für einen zweiten Spritzenkörper (10b) mit einem darin enthaltenem Fluid angeordnet ist und,
**dass** die zweite Aufnahme (8) mit der Unterdruckkammer (23) derart verbunden ist, dass sich das Fluid nach Abgabe aus einem in der zweiten Aufnahme (8) angeordneten zweitem Spritzenkörper (10b) innerhalb der Anlagefläche (13) auf dem Auge (3) verteilt.

2. Vorrichtung nach Anspruch 1, **dadurch gekennzeichnet, dass** die Unterdruckkammer (23) einen flexibel, manuell verformbaren Wandbereich (25) aufweist, wobei die Unterdruckkammer (23) derart ausgebildet ist, dass durch Verformung des Wandbereichs (25) das Volumen der Unterdruckkammer (23), ausgehend von einer ursprünglichen Größe, verkleinerbar ist und durch Rückverformung des Wandbereichs (25) das Volumen der Unterdruckkammer (23) die ursprüngliche Größe wieder annimmt.

3. Vorrichtung nach einem der vorhergehenden Ansprüche, **dadurch gekennzeichnet, dass** das Gehäuse (4) eine Verschiebeeinrichtung (15) aufweist, mittels der die erste Aufnahme (7) entlang einer Verschiebeachse (16), die sich von dem proximalen Ende (5) zum distalen Ende (6) des Gehäuse (4) hin erstreck, von einer Ruheposition in eine Injektionsposition verschiebbar geführt ist, wobei eine in der ersten Aufnahme (7) angeordnete Injektionsnadel (9) in der Ruheposition von dem Auge (3) beabstandet und in der Injektionsposition in das Auge (3) eingedrungen ist.

4. Vorrichtung nach Anspruch 3, **dadurch gekennzeichnet, dass** die Verschiebeeinrichtung (16) ein faltenbalgartige Struktur umfasst.

5. Vorrichtung nach Anspruch 4, **dadurch gekennzeichnet, dass** die faltenbalgartige Struktur als ziehharmonikaartiger Abschnitt (18) des Gehäuses (4) ausgebildet ist.

6. Vorrichtung nach Anspruch 3, **dadurch gekennzeichnet, dass** die Unterdruckkammer (23) an einer der Anlagenfläche (13) gegenüberliegenden Seite eine Durchgangsöffnung (26) aufweist, über die die Unterdruckkammer (23) mit der ersten Aufnahme (7) verbunden ist und, durch die die Injektionsnadel (9) in der Ruheposition der ersten Aufnahme (7) hindurchragt.

7. Vorrichtung nach einem der vorhergehenden Ansprüche, **dadurch gekennzeichnet, dass** das Gehäuse (4) einstückig geformt und vorzugsweise aus einem Elastomer hergestellt ist.

8. Vorrichtung nach einem der vorhergehenden Ansprüche, **dadurch gekennzeichnet, dass** das Gehäuse (4) an dem distalen Ende (6) eine Positionierungseinrichtung (27) zur Positionierung der Vorrichtung (2, 2') auf der Bindehaut (14) des Auges (3) aufweist.

9. Vorrichtung nach Anspruch 8, **dadurch gekennzeichnet, dass** die Positionierungseinrichtung (27) als Außenkontur (29) am distalen Ende (6) des Gehäuses (4) ausgebildet ist, wobei die Außenkontur (29) derart geformt ist, dass sie zumindest teilweise einer Außenkontur einer Iris (31) nachempfunden ist.

10. Vorrichtung nach Anspruch 8, **dadurch gekennzeichnet, dass** die Positionierungseinrichtung (27) als farbliche Markierung (28) des Gehäuses (4) ausgebildet ist.

11. Vorrichtung nach einem der vorhergehenden Ansprüche, **dadurch gekennzeichnet, dass** die Vorrichtung (2') einen Einsatz (35) aufweist, der in die erste Aufnahme (7) des Gehäuses (4) eingesetzt ist, wobei sich der Einsatz (35) ausgehend von dem proximalen Ende (5) zum distalen Ende (6) des Gehäuses (4) hin verjüngt.

12. Vorrichtung nach Anspruch 11, **dadurch gekennzeichnet, dass** die erste Aufnahme (7) eine umlaufende Wulst (36) umfasst, die ausgebildet ist, mit einer an dem Einsatz (35) vorgesehenen umlaufenden Nut (37) derart zusammenzuwirken, dass der Einsatz (35) in der ersten Aufnahme (7) fixiert ist.

13. Vorrichtung nach einem der vorhergehenden Ansprüche, **dadurch gekennzeichnet, dass** die Vorrichtung (2) weiter eine Injektionsnadel (9) umfasst, die in der ersten Aufnahme (7) angeordnet ist.

14. Injektionssystem umfassend:
eine Vorrichtung (2, 2') zur intravitrealen Injektion in ein Auge (3) nach einem der vorhergehenden Ansprüche 1 bis 13,
eine Injektionsnadel (9), die in der ersten Aufnahme (7) des Gehäuses (4) angeordnet ist,
einen ersten Spritzenkörper (10a), der mir der Injektionsnadel (9) verbunden ist, und
einen zweiten Spritzenkörper (10b), der in der zweiten Aufnahme (8) des Gehäuses (4) angeordnet ist.
